Europäisches Patentamt

(19) European Patent Office

Office européen des brevets

(11) Publication number : **0 409 842 B1**

(12) # EUROPEAN PATENT SPECIFICATION

(45) Date of publication of patent specification :
10.05.95 Bulletin 95/19

(51) Int. Cl.⁶ : **G01N 33/58,** G01N 33/28, G01N 33/94

(21) Application number : **89902404.6**

(22) Date of filing : **08.02.89**

(86) International application number :
**PCT/GB89/00121**

(87) International publication number :
**WO 89/07272 10.08.89 Gazette 89/18**

(54) **MARKING OF CHEMICAL PRODUCTS TO ESTABLISH IDENTITY AND SOURCE.**

(30) Priority : **08.02.88 GB 8802838**

(43) Date of publication of application :
**30.01.91 Bulletin 91/05**

(45) Publication of the grant of the patent :
**10.05.95 Bulletin 95/19**

(84) Designated Contracting States :
**AT BE CH DE FR GB IT LI LU NL SE**

(56) References cited :
EP-A- 117 648
EP-A- 256 551
CHEMICAL ABSTRACTS, vol. 106, no. 15, 13
April 1987, Columbus, OH (US); C.N. MARTIN
et al., p. 173, no. 114689u
CHEMICAL ABSTRACTS, vol. 100, no. 9, 27
February 1984, Columbus, OH US); J. RUP-
RICH et al., p. 475, no. 66700t

(73) Proprietor : **BIOCODE, INC.**
**P.O. Box 648**
**275 Mill Way**
**Barnstable, MA 02630 (US)**

(72) Inventor : **GARNER, Ronald Colin**
**5 Hall Drive**
**Sand Hutton, York (GB)**
Inventor : **MARTIN, Carl Nigel**
**Sandlands Cottage, Main Street**
**Thorganby, York (GB)**

(74) Representative : **Nicholls, Kathryn Margaret**
**MEWBURN ELLIS**
**York House**
**23 Kingsway**
**London WC2B 6HP (GB)**

## Description

### Technical Field

The present invention relates to the marking of chemical products to establish their identity and source. The products may, for example, be pharmaceuticals, food additives, cosmetics, agrochemicals, oil and petroleum products or other chemicals and mixtures of chemicals.

### Background Art

It is usually difficult to distinguish a chemical product having a particular chemical formula and made by one manufacturer, from the same chemical, with the same formula, but made by a different manufacturer. This is particularly so if the two manufacturers use the same production process. For this reason, it is not difficult for the unscrupulous to establish the chemical formula of an active ingredient in a composition, and the relative amounts of the various ingredients in the composition, and then pass off his own product as that of another manufacturer.

The present inventors have devised a method of labelling a chemical compound, or composition with a marker substance in such a way that the presence of the marker substance may be easily established by someone who knows the identity of the marker, but could not be routinely determined by a counterfeiter or other person unfamiliar with the marker. Thus, a counterfeit and a genuine product could be distinguished by the absence of the marker in the former and the presence of the marker in the latter.

The marker is also useful for indicating other information about the chemical, or chemical composition, such as its date of production, and, consequently, the end of its shelf-life.

WO 87/06383 discloses a method of labelling an item or substrate by means of a nucleic acid or proteinaceous marker. According to that document the marker susbtance is. in general, included on a tag, attached to the item to be marked and the tests described in the document determine the presence or absence of the marker without any quantification of the marker.

### Disclosure of the Invention

According to the present invention, however, there is provided a method for identifying the source of a chemical or chemical composition said method comprising

(a) adding one or more inert marker compounds to a chemical or chemical composition whose source is to be identified thereby to produce a marked chemical or marked chemical composition; subsequently

(b) binding any said at least one inert marker compound present in a test chemical or test chemical composition, whose source it is desired to establish, to a respective complementary binding member for the or each inert marker compound to form an immunological binding pair, thereby to concentrate the or each marker compound; and

(c) determining the concentration of the or each marker compound in the test chemical or test chemical composition by subjecting the or each bound marker to analysis; and

(d) comparing the result of step (c) with that obtainable when the same concentrating and analysis steps are carried out on the marked chemical or marked chemical composition.

Any suitable analytical technique may be used for the analysis, for example immunoassay or high performance liquid chromatography (HPLC).

The inert marker compound should, in general, be one which is not normally present in the chemical or composition; for example it is not a by-product of the production process, normal impurity, or standard additive for that chemical, or chemical composition. The marker compound is present in very low concentrations, in the order of micrograms per kilogram of the labelled composition or below. Although it is inert in the sense that it does not react with the chemical which it labels, it must nevertheless be capable of binding to a complementary binding member. For example, the marker is suitably a molecule capable of being bound by an antibody, preferably a monoclonal antibody for it.

The present invention facilitates the identification of several different batches of a chemical or chemical composition by the use of a single marker compound. This is because a single marker compound may be employed in different concentrations in different batches and each batch identified by determination of the concentration of the marker in that batch.

In preferred embodiments a plurality of marker compounds are included in a chemical or composition. In this case the number of possible permutations of concentration and markers is increased and batches may be identified with increased certainty by measuring relative concentrations of the markers.

2

In some embodiments of the present invention the or each marker substance is extracted from the chemical or composition into a solvent before binding to its complementary binding member. The solvent is preferably one which is compatible with the complementary binding member. Alternatively, where the extraction solvent is incompatible with the complementary binding member the extract may be diluted with a compatible solvent before binding to the complementary binding member.

The complementary binding member is preferably an antibody and particularly preferably a monoclonal antibody. The complementary binding member or members are desirably provided on an immunoaffinity column.

The marked chemical or chemical composition preferably contains at least one inert marker compound, the or each marker compound being present in a concentration of not more than 5 ppm by weight. Particularly preferably, the or each marker is present in a concentration of not more than 100 parts per billion by weight.

It will be apparent from the above discussion that preferred marker compounds are ones which are capable of binding to a corresponding antibody. Preferably, they are small organic molecules which are haptenic; that is molecules to which antibodies may be generated by administering them in association with a carrier molecule.

Preferably several markers are included in a chemical or composition. The ratios of the concentrations of the markers in each chemical or composition labelled are then preferably unit ratios, e.g. in the case where there are two markers the ratio of concentration of one to that of the other may be 1:1, 1:2, 1:3, 1:4, etc.

Preferably, where a plurality of markers are present these possess a common site which enables then to bind to, and be concentrated on the same complementary binding member, while they remain separable by subsequent analytical techniques. Thus in some embodiments at least one of the marker Compounds may carry an amino acid, nucleic acid, oligonucleotide or oligopeptide substituent which does not affect the binding of the marker compound to its complementary binding member. Such substituents make marker compounds more easily distinguishable from each other by analytical techniques such a hplc.

Although a variety of substances may be labelled the chemical or composition containing a marker compound is preferably a high added value product such as a food additive, pharmaceutical or cosmetic.

The method of the present invention may be carried out using a kit for labelling a chemical or chemical composition and for identifying a predetermined marker substance. The kit preferably comprises at least one inert marker compound and at least one complementary binding member for said at least one inert marker compound which binds the marker to form an immunological binding pair.

It will be apparent that, since the marker compound is in such low concentrations in the labelled chemical or composition its presence therein is not immediately apparent to someone who is unaware of the addition. Furthermore, it would not be easy for a third party to identify the marker using routine techniques and include it in a counterfeit composition. That is because isolation and concentration of the marker relies on the use of an antibody specific for it and this would not be available to anyone who was ignorant of the identity of the marker.

Brief Description of the Drawings

Embodiments of the invention are described below by way of example with reference to the accompanying figures of which

Fig. 1 shows the formulae of fungal metabolites which can be employed as markers

Fig. 2 shows inhibition ELISA results for anti-resorcyclic acid lactone (RAL) monoclonal antibody and anti-aflatoxin antibody

Fig. 3A shows the isocratic elution profile of 25 $\mu$l of a standard solution containing 200 ng/ml of each of zearalenol (ii), zearalanol (iii) and zearalenone (iv)

Fig. 3B shows the elution profile of the same compounds after extraction from paracetamol

Figs. 4A shows the elution profile of N-acetyl-L-lysine aflatoxin B[1] (250$\mu$l of 300ng/ml)

Figs 4B and 4C show the elution profiles of N-acetyl-L-lysine aflatoxin $B_1$ as extracted from aftershave by two different methods.

Modes for carrying out the Invention

In the examples members of two separate groups of fungal metabolites are used as examples of small organic molecules which may be used at very low concentrations to label a substrate and which may be extracted specifically by immunoaffinity chromatography and quantified in order to allow identification of both the source and batch of that substrate. Although the fungal metabolites employed in these examples are toxins the results obtained indicate that the technique could be extended so that pharmaceuticals and food additives

EP 0 409 842 B1

could be labelled with other small molecules which present no risk to human health.

Two monoclonal antibodies directed against the two separate groups of fungal metabolites are used in the examples. The first, directed against resorcyclic acid lactones (RALs), reacts with the three molecules zearalenone, zearalenol and zearalanol which are readily separated by high performance liquid chromatography (HPLC) . The second antibody reacts with several aflatoxins (the other group of fungal metabolites). These aflatoxins may also be separated by HPLC.

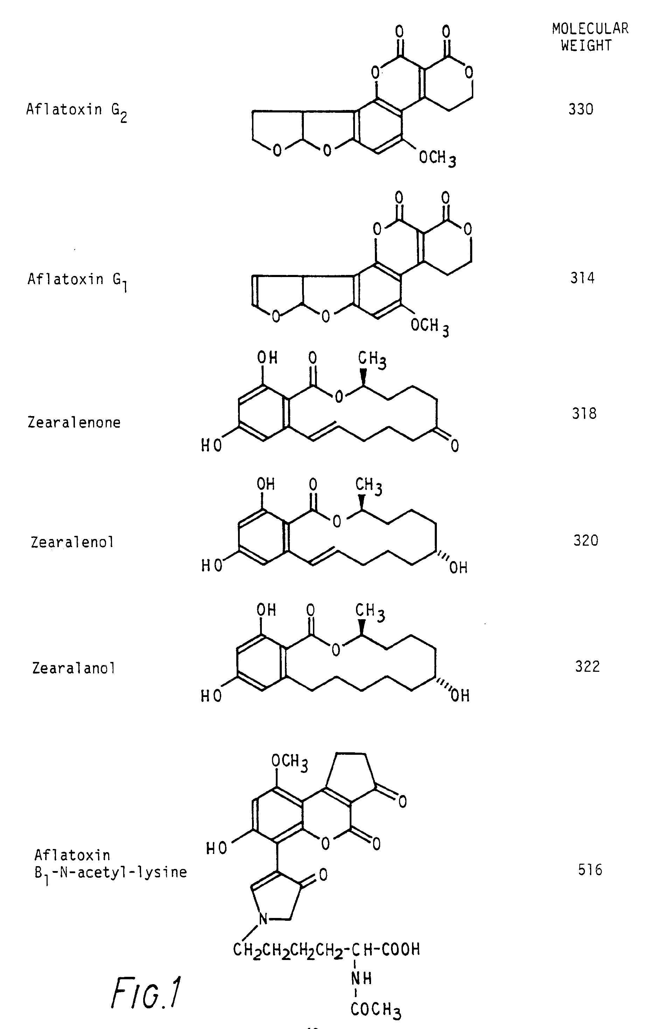

Each monoclonal antibody reacts with several similar fungal metabolites because the antigen binding sites on the antibody are directed towards a chemical species which is common between all fungal metabolites of a particular group. It is also possible to derivatize certain parts of the metabolites without hindering their binding to the antibody. For example, N-acetyllysine aflatoxin $B_1$ is still bound by the anti-aflatoxin antibody. The structures of the small organic molecules used in this study are shown in Figure 1.

It is possible to use amino acids as building blocks to generate an enormous variety of potential marker chemicals with which to mark high added value products. As a model the applicants have therefore conjugated one amino acid derivative, called N-acetyllysine to aflatoxin $B_1$ to exemplify this procedure, and demonstrated the use of this derivative in the marking of a cosmetic product.

MATERIALS AND METHODS

A. Conjugation Reactions

A(i) Preparation of N-acetyllsine-aflatoxin $B_1$

Aflatoxin $B_1$ (16.7 mg) was dissolved in dichloromethane (1 ml). Chlorine gas was bubbled through the solution for 5 minutes followed by evaporation under vacuum. The resultant mixture of compounds including 8, 9-dihydro-8, 9-dichloro-aflatoxin $B_1$, was dissolved in 0.6 ml of dimethysulphoxide (DMSO) and mixed with 500 mg of N-acetyllysine dissolved in 20 ml of 0.1 M phosphate buffer (pH 7.4). The reagents were mixed for 48 hours at room temperature followed by high performance liquid chromatography (hplc) purification on an S5-ODS 2 column eluted with an acetonitrile in water gradient as follows:

| Time | % acetonitrile: water (v/v) |
|------|------|
| 0 | 0 |
| 6 | 30 |
| 12 | 100 |
| 15 | 100 |
| 17 | 0 |
| 27 | 0 |

A (ii) Preparation of Aflatoxin - Protein Conjugates

Briefly, aflatoxin $B_1$-ovalbumin was prepared for inoculation of mice (see below) by direct chlorination of aflatoxin $B_1$ to give 8,9-dihydro-8, 9-dichloro-aflatoxin $B_1$, (Sabbioni et al, 1987), followed by reaction with ovalbumin in 0.1 M phosphate buffer (pH 7.4) and dialysis. The resultant antigen consisted of 10 to 15 molecules of aflatoxin $B_1$ covalently coupled to ovalbumin.

Aflatoxin $B_1$ was coupled to bovine serum albumin (BSA) in the same way for coating of ELISA plates (see below).

A(iii) Preparation of Zearalenone-Protein Conjugates

Briefly, zearalenone-ovalbumin was prepared for inoculation of mice by carboxylation of hydroxyl groups on zearalenone with carboxymethoxyalanine followed by reaction between the new carboxyl group and amino groups on the protein by carbodiimide reaction (Thouvenot and Morfin, 1983). The resultant antigen consisted

4

of 10 to 15 molecules of zearalenone covalently linked to 1 molecule of ovalbumin.

Zearalenone was also coupled to bovine serum albumin (BSA) in the same way for coating of ELISA plates.

## B Preparation of Monoclonal Antobodies

### B(i) Inoculation of mice

Antigens (prepared as protein conjugates as described above) were prepared as 1 mg/ml solutions in a 50% (v/v) emulsion of Freund's complete adjuvant in phosphate buffered saline. This emulsion was inoculated into the peritoneal cavity of 10 C57 black x BALB-C f-1 female mice. Several inoculations (300 μg antigen/inoculation) were performed at approximately 3-weekly intervals followed by sino-orbital bleeding of the mice. Antiserum levels were monitored by indirect and indirect-inhibition enzyme linked immunosorbent assays (ELISA) for antibodies with specific binding of aflatoxin/zearalenone.

### B(ii) Mouse Spleen Fusion Technique

HT medium consisted of RPMI medium with L-glutamine (Gibco Europe Ltd) containing (20% v/v) foetal calf serum, 0.05 mM 2-mercaptoethanol, 1 mM sodium pyruvate, 100 mM hypoxanthine, 100 mM thymidine, 100 μg/ml gentamycin and 2.5 μg/ml fungizone. X63 myeloma cells (Flow Laboratories, UK) were grown in HT medium with the hypoxanthine and thymidine replaced by 8-azaguanine (100 mM). Spleen cells from one immunized mouse spleen ($10^8$) were fused with X63 myeloma cells ($5 \times 10^6$ in log phase) using GC grade polyethylene glycol 4000 (Merck) 50% w/v in serum-free medium containing 20% (v/v) dimethyl sulphoxide (DMSO) added slowly over 60 seconds, followed by 90 seconds standing at room temperature. The mixture was gradually diluted with 10 ml GKN (8g NaCl, 0.4 g KCl, 1.42 g $Na_2HPO_4$, 0.78 g $NaH_2PO_4.2H_2O$, 2 g glucose, 0.1 g phenol red in 1L $H_2O$) over 5 minutes, followed by standing at room temperature for 10 minutes. Cells were distributed into 10 x 96-well Costar® tissue culture plates (Northumbria Biologicals, UK), containing approximately $10^4$ mouse peritoneal macrophages per well, and hybridomas selected in HAT medium (HT medium containing aminopterin). After approximately two weeks, supernatants were screened for specific antibody production by enzyme-linked immunosorbent assay (ELISA) and specificity confirmed by inhibition ELISA as described below. Positive hybridomas were cloned by limiting dilution.

### B(iii) ELISA Techniques

Solutions and buffers. PBS (11.3 mM KCl, 0.7 mM $KH_2PO_4$, 8.2 mM $Na_2HPO_4$, 8.2 mM $Na_2HPO_4$, 138 mM NaCl, pH 7.2). Solution A: PBS containing 1.5 mM $MgCl_2$, 2 mM 2-mercaptoethanol, 0.05% (v/v) Tween 20. Solution B: 10 mM $K_2HPO_4$, 150 mM NaCl, 1% w/v bovine serum albumin, pH 7.2. Solution C: 3, $3^1$, 5, $5^1$-Tetramethylbenzidine (100 μg/ml) freshly prepared in 100 mM trisodium citrate, 100 mM sodium acetate, (pH 6.0) with $H_2O_2$ (20 μl of 100 volumes per 100 ml of solution).

Techniques Indirect ELISA and inhibition ELISA techniques were used as previously described (Voller et al, 1979) to screen and quantify the monoclonal antibodies. Five modifications were made to these procedures: (i) Either aflatoxin $B_1$-bovine serum albumin, or zearalenone-bovine serum albumin antigens (50 ng/well) is PBS were dried at 37 C for 24 hours onto clear polyvinyl chloride 96-well Costar® microtitre plates (Northumbria Biologicals, UK) during coating; (ii) solution A was used for washing plates (four times) between incubation steps; (iii) first and second antibodies were diluted in solution B and incubated on the plates for 1 hour at room temperature with shaking; (iv) for a competitive inhibition ELISA, mixtures of antigen and antibodies were incubated for 1 hour at room temperature on the plate (total volume of 75 μl) prior to washing and subsequent steps; (v) prior to incubation with substrate (solution C) plates were washed six times with solution A and twice with distilled water. Fifteen minutes after incubation with substrate the reaction was stopped with an equal volume of 2M $H_2SO_4$.

## C Preparation of Immunoaffinity Columns

An immunoaffinity resin was prepared by reacting cyanogen bromide Sepharose® CL-4B resin (Pharmacia Ltd) with monoclonal antibody (ratio 1 ml resin to 2.5 mg of antibody). Cyanogen bromide Sepharose® CL-4B was activated by swelling in 75 ml/g of 1 mM HCl. The resin was then washed with 5 ml/g of coupling buffer (100 mM sodium hydrogen carbonate, 500 mM sodium chloride, pH 8.3). Antibody was diluted to 1.56 mg/ml with PBS followed by diluting with an equal volume of coupling buffer. This mixture was reacted with the swelled, washed resin for 1 hour at room temperature with mixing. The resin was filtered and mixed with ethanolamine (1M at pH 8.0) for 1 hour at room temperature. The resin was washed and stored with PBS containing 0.05% w/v sodium azide at pH 7.4. Aliquots of the finished resin (0.04 ml settled bed volume) were then supported between porous frits in plastic columns. These columns were examined for their ability to bind mycotoxin standards when these were applied in 50 ml of PBS and eluted with acetonitrile (2 ml). Quantification was by HPLC analysis.

## D Extraction Methods

### D(i) Extraction of Resorcyclic acid lactones (RALS) from Pharmaceutical Products

Concentrations of standard RAL solutions were determined from absorbance determinations at lambda max, the extinction coefficient for the particular RAL and its molecular weight. Aliquots of ground paracetamol tablets (6 g per aliquot) were spiked with 2.4 ug of each of the RALS, zearalenone, zearalenol and zearalanol, in different combinations as shown in Table 1. Each sample was then extracted by blending with 12 ml of 30% (v/v) acetonitrile: water for 2 minutes. Each sample was centrifuged at 1400 g (av) for 2 minutes and 4 ml was removed and diluted to 40 ml with distilled water prior to immunoaffinity chromatography on columns prepared in section 2.C. The column was washed with 10 ml of distilled water prior to elution with 2 ml of acetonitrile; 2 ml of hplc grade water was added and the volume measured.

Samples (250 μl) were chromatographed on an S5 ODS 2 HPLC column by isocratic elution (50% (v/v) acetonitrile : water) and absorbance was monitored at 236 nm. Standards of comparable peak area were chromatographed before and after repeat sample runs in order to quantify the concentration of RALs occurring in each eluent.

### D(ii) Extraction of aflatoxins from pharmaceutical products

The concentrations of aflatoxin $G_1$ and $G_2$ standards were determined spectrophotometrically and 200 ng of each were used to spike 10 g of ground paracetamol follwed by blending for 2 minutes with 20 ml of 30% (v/v) acetonitrile : water. This was then centrifuged at 1400 g (av) for 2 minutes. 4 ml of supernatant was removed and added to 44 ml of PBS, followed by immunoaffinity chromatograpy as described for RALS. Eluted aflatoxins were chromatographed on an S5 ODS 2 column isocratically eluted with 40% (v/v) of (5.4 v/v) acetonitrile : methanol) in water with post column derivatization of the aflatoxins with saturated aqueous iodine solution prior to fluorescence monitoring. Standards were chromatographed before and after repeat sample runs in order to quantify the concentration of aflatoxins in the eluent.

### D(iii) Extraction of Derivatized Lysine from Cosmetics

N-acetyl Lysine-Aflatoxin $B_1$ was dissolved in water at 1 mg/ml by weight, and this stock solution was used to spike Aramis® aftershave which was extracted using two methods.

Method A:

N-acetyl-lysine-aflatoxin $B_1$ (2 ug) was spiked into 2 ml of Aramis® aftershave. After approximately 30 minutes HPLC water (1 ml) was added and the mixture rotary evaporated to a volume of approximately 1 ml. This was diluted with 50 ml of PBS followed by immunoaffinity chromatography. N-acetyl lysine aflatoxin $B_1$ that bound to the column was eluted with 2:1 (v/v) acetonitrile : water followed by rotary evaporation to approximately 600 ul. This sample was diluted with HPLC water prior to chromatography through an S5 ODS 2HPLC column using a gradient as described in section A(i) for elution. peak areas of samples were compared with peak areas of standards of known concentration for quantification of N-acetyl-lysine-aflatoxin $B_1$.

Method B:

N-acetyl-lysine-aflatoxin $B_1$ (500 g) was spiked into 0.5 ml of Aramis aftershave. The mixture was diluted to 50 ml with PBS followed by immunoaffinity chromatography. Elution and HPLC analysis were as described for Method A.

RESULTS AND DISCUSSION

A Specificity of monoclonal antibodies

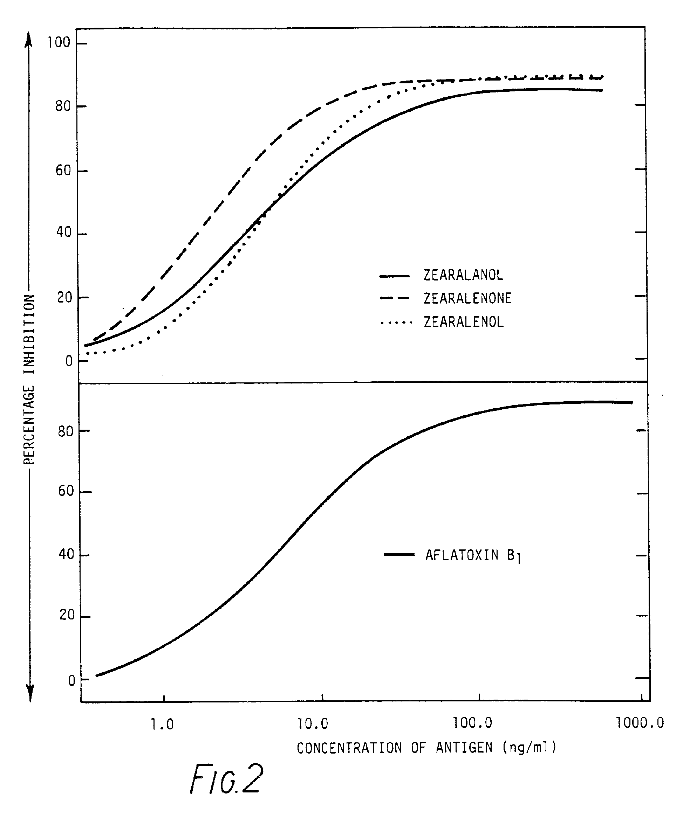

Several monoclonal antibody producing hybridoma cell lines were produced (as in section B above) against each group of fungal metabolites. Productivity of these cell lines was established by indirect ELISA of supernatant medium after cells had been grown to death, and, a measure of sensitivity and specificity of these monoclonal antibodies was determined by inhibition ELISA (B(iii) above). Inhibition ELISA results for the selected anti-RAL monoclonal antibody and anti-aflatoxin antibody are shown in Figure 2. Both were selected on the basis of their 50 percent inhibitor concentration ($IC_{50}$) values which are both in the picomolar range and on this basis should enable the production of efficient immunoaffinity resins.

B Capacities of Immunoaffinity Columns for Binding RALS and Aflatoxins

Specific immunoaffinity columns prepared as in section C above were examined for their ability to bind increasing amounts of fungal metabolites from PBS.

Anti-RAL immunoaffinity columns had a capacity for binding in excess of 7.0 μg of zearalenone from 50 ml of PBS. They bound between 85 and 95% of up to 5000 ng of RALs applied in 50 ml of PBS.

Anti-aflatoxin immunoaffinity columns bound in excess of 2 μg of aflatoxin $B_1$ from 50 ml of PBS. They consistently bound in excess of 90% of up to 1000 ng of either aflatoxin $B_1$, $B_2$, $G_1$ or $G_2$.

The quantity of aflatoxin bound to immunoaffinity columns does not need to be as large as that of RALs since aflatoxins may be detected at much lower levels than RALs. The next section shows how these immunoaffinity columns were used to extract derivatives of fungal metabolites from two high added value products.

C The Use of Small Organic Molecules to Identify Products

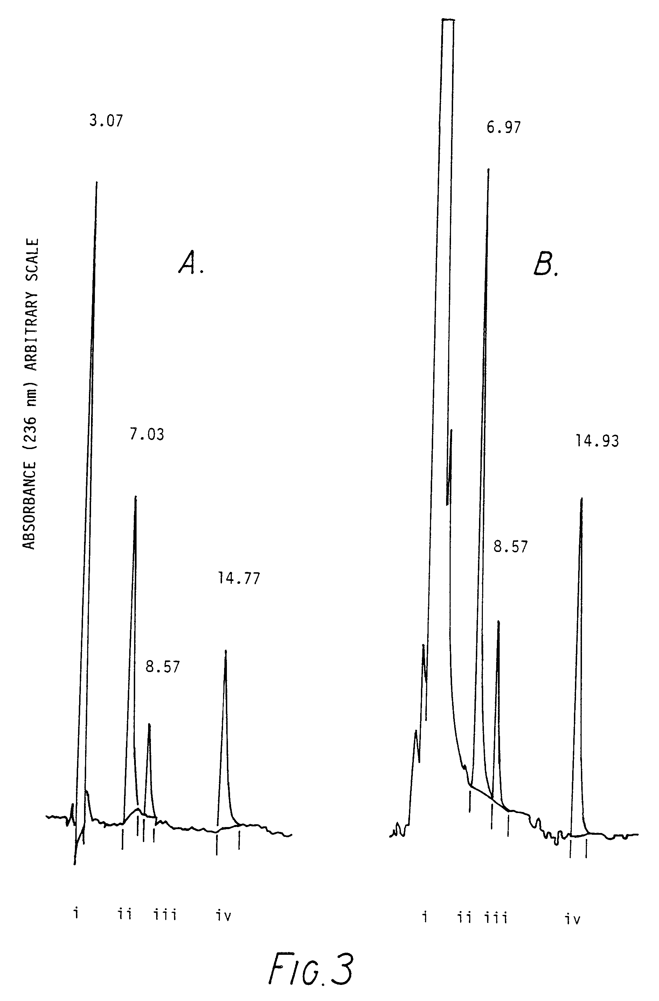

Three RALs (400 ng/g) and two aflatoxins (20 ng/g) spiked into paracetamol tablets in different combinations were extracted with greater than 70% efficiency from this pharmaceutical, (Table 1). Each fungal metabolite was quantified from the mean of three sample determinations of peak area compared with peak areas of standard solututions chromatographed before and after the samples. For example, figure 3A shows the isocratic elution profile of 250 μl of a standard solution containing 200 ng/ml of each of zearalenol (ii), zearalanol (iii) and zearalenone (iv). Figure 3B shows the elution profile of the RALs after extraction from paracetamol as described in section D (i) above. Some polar material (peak (i)) contaminates the extract, however, this is of no relevance since it does not interfere with quantification of RALs. Retention times (minutes) are given at the top of integrated peaks. Different combinations of RALs and aflatoxins used as spikes can therefore be used to distinguish between different batches of products. Extraction of these fungal metabolites occurred in two phases.

Phase 1. The success of the initial solvent extraction from paracetamol was dependent not only on the solubility of the fungal metabolite in the 30% (v/v) acetonitrile : water but also on the solubility of other chemical constituents of paracetamol. Extraction in this phase was found to be optimal in 30% (v/v) acetonitrile : water when compared with 10, 50 and 70% (v/v).

Phase 2. The second phase of extraction involved application of the first extract to the immunoaffinity column. First the solvent was diluted in aqueous solution to a level at which antibodies were able to bind antigen (as organic solvents destroy this ability). Solubility of the fungal metabolite at this stage affects its binding to the immunoaffinity column.

Aflatoxin extracts were diluted to less than 2.5% (v/v) acetonitrile : PBS and RAL extracts to less than 10% (v/v) acetonitrile : water prior to application to the columns.

The efficiency of the eluent for removal of the antigen from the immunoaffinity resin is again dependent on the solubility of the antigen in the eluent, but also on the ability of the eluent to denature the antibody. The latter is dependent on a host of effects. Probably the most important of these effects are factors affecting hydration of the antibody in the eluent solution. The optimum eluent was found to be 100% acetonitrile. This gave satisfactory recoveries of both RALs and aflatoxins and could be diluted to 50% (v/v) acetonitrile in HPLC water for direct quantification by HPLC.

It is likely that the nature of extraction solvents and eluent solvents would be dependent on the nature of the chemicals chosen for spiking individual substrates. However, the results described here show that it is possible to add low concentrations of small organic molecules to pharmaceutical products and to use immunoaffinity chromatography to identify and distinguish between batches of the product.

## D The Use of Amino Acids as Building Blocks

As an extension of the ability to use small organic molecules for the purpose of identifying and distinguishing between batches of high added value products it is possible to conjugate numbers of different amino acids to a given small organic molecule to which antibodies have been made. Whilst this does not alter the immunoaffinity chromatography of these conjugated small organic molecules it renders them more easily separable by a detection method such a hplc. Indeed, by hplc, different amino acid(s) and/or different sequences of the same amino acids could be easily separated. The number of possible combinations is very large as there are 20 natural amino acids and many synthetic chemicals which can be used in such a process. Selection of conjugates depends on their solubility in the solvents used for immunoaffinity chromatography as described in section C of "Results and Discussion".

As a model for this procedure, the applicants have conjugated N-acetyl-lysine to aflatoxin $B_1$ and investigated the conditions for extraction of this from after-shave using immunoaffinity columns directed against aflatoxin. The conjugation was performed as in section (A (i) of Materials and Methods) and confirmed as N-acetyl-L-lysine aflatoxin $B_1$ spectrophtometrically. This conjugate is readily extractable from liquid cosmetics. For example, Aramis® aftershave (500 ul) spiked with N-acetyl-L-lysine aflatoxin $B_1$ (500 ng) was either (a) rotary evaporated or (b) diluted to 1% (v/v) with PBS to lower the alcohol content of this product. This allowed the immunoaffinity column to bind the conjugate which was selectively eluted by applying 67% (v/v) acetonitrile HPLC grade water to the column. Acetonitrile (non-aqueous) is not an effective eluent and gave very poor yield since the conjugate is less soluble in this than in aqueous acetonitrile.

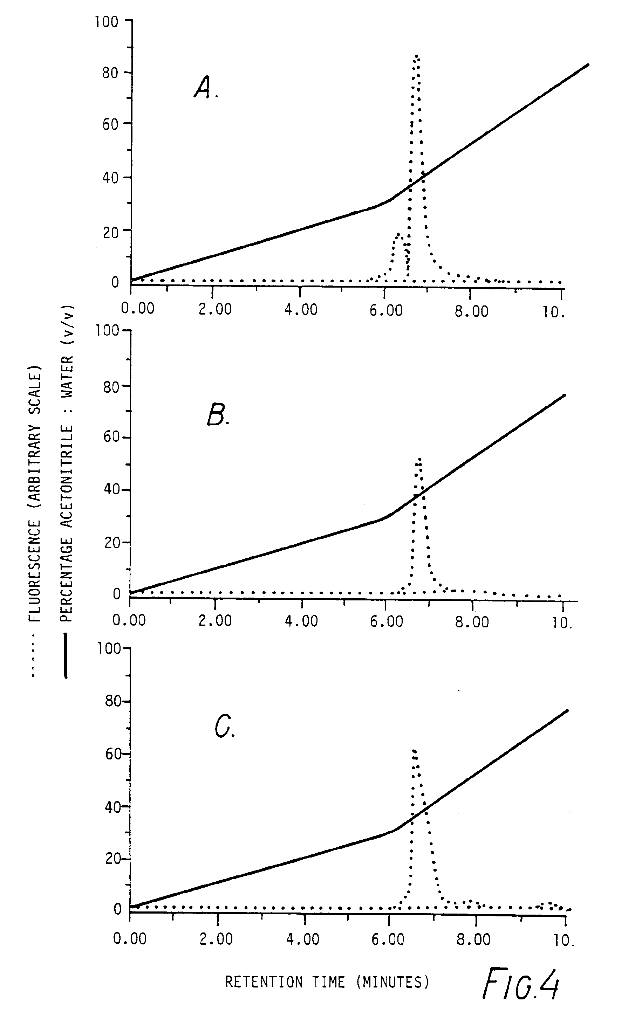

Figure 4 shows the elution profile of N-acetyl-L-lysine-aflatoxin $B_1$ (250 ul of 300 ng/ml) prepared as in section A (i) of "Materials and Methods" and chromatographed under conditions as described in section D (iii) of "Materials and Methods". The elution gradient (y-axis) is overlaid on the profile, with elution time in minutes on the X-axis. Figure 4B shows the result of using method A (section D (iii) of Materials and Methods) for extraction from Aramis® aftershave (ie initial rotary evaporation to remove alcohol). Had the extraction been 100% efficient this sample would contain 242 ng/ml of N-acetyl-L-lysine-aflatoxin $B_1$. The actual efficiency was 79%. Figure 4C shows the result of using method B (section D (iii) of Materials and Methods) for extraction from Aramis® (ie direct dilution of aftershave). Had the extraction been 100% efficient this sample would contain 325 ng/ml of N-acetyl-L-lysine aflatoxin $B_1$. The actual efficiency was 73%. Aramis® which did not contain the N-acetyl-L-lysine aflatoxin $B_1$ marker showed no chromatographed peak during elution.

EP 0 409 842 B1

TABLE 1.    EXTRACTION OF SMALL ORGANIC MOLECULES FROM PARACETAMOL

| | ZEARALENOL | | | ZEARALANOL | | | ZEARALENONE | | | AFLATOXIN $G_1$ | | | AFLATOXIN $G_2$ | | |
|---|---|---|---|---|---|---|---|---|---|---|---|---|---|---|---|
| | Amount Added ng/g | Amount Recovered ng/g | % Recovery | Amount Added ng/g | Amount Recovered ng/g | % Recovery | Amount Added ng/g | Amount Recovered ng/g | % Recovery | Amount Added ng/g | Amount Recovered ng/g | % Recovery | Amount Added ng/g | Amount Recovered ng/g | % Recovery |
| 1 | 400 | 344 | 86 | 400 | 340 | 85 | 400 | 356 | 89 | 0 | 0 | 0 | 0 | 0 | 0 |
| 2 | 400 | 280 | 70 | 0 | 0 | 0 | 400 | 380 | 95 | 0 | 0 | 0 | 0 | 0 | 0 |
| 3 | 400 | 288 | 72 | 400 | 404 | 101 | 0 | 0 | 0 | 0 | 0 | 0 | 0 | 0 | 0 |
| 4 | 0 | 0 | 0 | 400 | 396 | 99 | 400 | 412 | 103 | 0 | 0 | 0 | 0 | 0 | 0 |
| 5 | 0 | 0 | 0 | 0 | 0 | 0 | 400 | 388 | 97 | 0 | 0 | 0 | 0 | 0 | 0 |
| 6 | 400 | 356 | 89 | 0 | 0 | 0 | 0 | 0 | 0 | 0 | 0 | 0 | 0 | 0 | 0 |
| 7 | 0 | 0 | 0 | 400 | 320 | 80 | 0 | 0 | 0 | 0 | 0 | 0 | 0 | 0 | 0 |
| 8 | 0 | 0 | 0 | 400 | 320 | 80 | 400 | 400 | 100 | 0 | 0 | 0 | 0 | 0 | 0 |
| 9 | 0 | 0 | 0 | 0 | 0 | 0 | 0 | 0 | 0 | 20 | 15.5 | 76.3 | 20 | 16 | 80.0 |

REFERENCE LIST

1. Sabboni G, Skipper P L, Buchi G, Tannenbaum S R (1987). Isolation and Characterization of the Major Serum Albumin Adduct Formed by Aflatoxin $B_1$ in vivo in rats. Carcinogenesis, 8 (6), 819-824.

2. Thouvenot D and Morfin R F (1983). Radioimmunoassay for Zearalenone and Zearalenol in Human Serum. Applied Environ. Microbiol., 45 (1), 16-23.

3. Voller A, Bidwell D E Bartlett A (1979). The Enzyme Linked Immunosorbent Assay (ELISA). A Guide With Abstracts of Microplate Applications Available from Dynatech Europe, Borough House, Rue du Pre, Guernsey.

**Claims**

1. A method for use in identifying the source of a chemical or chemical composition, the method comprising:

    (a) adding one or more inert marker compounds to a chemical or chemical composition whose source is to be identified thereby to produce a marked chemical or marked chemical composition; subsequently

    (b) binding any said at least one inert marker compound present in a test chemical or test chemical composition, whose source it is desired to establish, to a respective complementary binding member for the or each inert marker compound to form an immunological binding pair, thereby to concentrate the or each marker compound; and

    (c) determining the concentration of the or each marker compound in the test chemical or test chemical composition by subjecting the or each concentrated marker to analysis.

2. A method according to claim 1 wherein the chemical or chemical composition is a pharmaceutical, food additive, cosmetic or agrochemical.

3. A method according to claim 1 or claim 2 wherein the or each marker compound is added to give a concentration of not more than 5 ppm by weight.

4. A method according to claim 3 wherein the concentration of the or each said marker is not more than 100 parts per billion by weight.

5. A method according to any preceding claim wherein a plurality of marker compounds are added in said step (a).

6. A method according to claim 5 wherein each of said marker compounds is capable of binding to the same complementary binding member and this is used for said binding step (b).

7. A method according to any preceding claim wherein, in step (b) the complementary binding member of the or each marker is a corresponding antibody.

8. A method according to any one of the preceding claims wherein step (a) comprises the addition of at least one marker compound which has an amino acid, nucleic acid, oligopeptide or oligonucleotide substituent which does not affect the binding of the marker compound to its complementary binding member.

9. A method according to any preceding claim wherein the or each marker is extracted from said chemical or composition before binding to its complementary binding member.

10. A method according to any preceding claim wherein the or each inert marker compound is bound by its respective complementary binding member present on an immunoaffinity column.

11. A method according to any preceding claim wherein each respective complementary binding member is a monoclonal antibody specific for its inert marker compound.

**Patentansprüche**

1. Verfahren zur Verwendung beim Identifizieren der Herkunft einer Chemikalie oder chemischen Zusam-

EP 0 409 842 B1

mensetzung, wobei das Verfahren umfaßt:

(a) Zugabe einer oder mehrerer Markerverbindungen zu einer Chemikalie oder chemischen Zusammensetzung, deren Herkunft zu bestimmen ist, um dadurch eine markierte Chemikalie oder markierte chemische Zusammensetzung herzustellen; anschließend

(b) Binden jeglicher zumindest einen inerten Markerverbindung, die in einer Testchemikalie oder chemischen Testzusammensetzung vorhanden ist, deren Herkunft bestimmt werden soll, an ein komplementäres Bindeglied für die oder jede Markerverbindung, um ein immunologisches Bindungspaar zu bilden, um dadurch die oder jede Markerverbindung zu konzentrieren; und

(c) Bestimmen der Konzentration der oder jeder Markerverbindung in der Testchemikalie oder chemischen Testzusammensetzung durch Analyse der oder jeder konzentrierten Markerverbindung.

2. Verfahren nach Anspruch 1, worin die Chemikalie oder chemische Zusammensetzung ein Pharmazeutikum, Nahrungsmittelzusatz, Kosmetikum oder eine landwirtschaftliche Chemikalie ist.

3. Verfahren nach Anspruch 1 oder 2, worin die oder jede Markerverbindung zugegeben wird, um eine Konzentration von nicht mehr als 5 Gew.-ppm zu ergeben.

4. Verfahren nach Anspruch 3, worin die Konzentration des oder jedes Markers nicht mehr als 100 Gew.-Teile pro Milliarde beträgt.

5. Verfahren nach irgendeinem der vorangegangenen Ansprüche, worin eine Vielzahl von Markerverbindungen im Schritt (a) zugegeben wird.

6. Verfahren nach Anspruch 5, worin jede der Markerverbindungen fähig ist, sich an dasselbe komplementäre Bindeglied zu binden, und dieses für den Bindungsschritt (b) verwendet wird.

7. Verfahren nach irgendeinem der vorangegangenen Ansprüche, worin in Schritt (b) das komplementäre Bindeglied für den oder jeden Marker ein korrespondierender Antikörper ist.

8. Verfahren nach irgendeinem der vorangegangenen Ansprüche, worin Schritt (a) die Zugabe von zumindest einer Markerverbindung umfaßt, die einen Aminosäure-, Nukleinsäure-, Oligopeptid- oder Oligonukleotid-Substituenten aufweist, der die Bindung der Markerverbindung an ihr komplementäres Bindeglied nicht beeinflußt.

9. Verfahren nach irgendeinem der vorangegangenen Ansprüche, worin der oder jeder Marker aus der Chemikalie oder Zusammensetzung vor dem Binden an sein komplementäres Bindeglied extrahiert wird.

10. Verfahren nach irgendeinem der vorangegangenen Ansprüche, worin die oder jede inerte Markerverbindung durch ihr entsprechendes komplementäres Bindeglied gebunden wird, das auf einer Immunoaffinitäts-Säule vorliegt.

11. Verfahren nach irgendeinem der vorangegangenen Ansprüche, worin jedes entsprechende komplementäre Bindeglied ein monoklonaler Antikörper ist, der für seine inerte Markerverbindung spezifisch ist.


**Revendications**

1. Méthode pour utilisation dans l'identification de la source d'un produit chimique ou d'une composition chimique, la méthode comprenant :

(a) l'ajout d'un ou plus composés marqueurs inertes à un produit chimique ou une composition chimique dont la source est à identifier pour produire par là un produit chimique marqué ou une composition chimique marquée; ensuite

(b) la liaison de l'un quelconque dudit au moins un composé marqueur inerte présent dans un produit chimique test ou dans une composition chimique test, dont on désire établir la source, à un membre de liaison complémentaire respectif pour le ou chaque composé marqueur inerte pour former une paire de liaison immunologique, pour concentrer par là le ou chaque composé marqueur; et

(c) la détermination de la concentration du ou de chaque composé marqueur dans le produit chimique test ou la composition chimique test en soumettant le ou chaque marqueur concentré à une analyse.

11

2. Méthode selon la revendication 1 où le produit chimique ou la composition chimique est un produit pharmaceutique, un additif alimentaire, un produit agrochimique ou cosmétique.

3. Méthode selon la revendication 1 ou la revendication 2 dans laquelle le ou chaque composé marqueur est ajouté pour donner une concentration de pas plus de 5 ppm en poids.

4. Méthode selon la revendication 3 dans laquelle la concentration du ou de chaque dit marqueur n'est pas supérieure à 100 parties par billion en poids.

5. Méthode selon l'une quelconque des revendications précédentes dans laquelle une pluralité de composés marqueurs sont ajoutés dans ladite étape (a).

6. Méthode selon la revendication 5 dans laquelle chacun desdits composés marqueurs est capable de liaison au même membre de liaison complémentaire et ceci est utilisé pour ladite étape (b) de liaison.

7. Méthode selon l'une quelconque des revendications précédentes dans laquelle, à l'étape (b) le membre de liaison complémentaire du ou de chaque marqueur est un anticorps correspondant.

8. Méthode selon l'une quelconque des revendications précédentes dans laquelle l'étape (a) comprend l'addition d'au moins un composé marqueur qui a un substituant acide aminé, acide nucléique, oligopeptide ou oligonucléotide qui n'affecte pas la liaison du composé marqueur à son membre de liaison complémentaire.

9. Méthode selon l'une quelconque des revendications précédentes dans laquelle le ou chaque marqueur est extrait dudit produit chimique ou de ladite composition chimique avant liaison à son membre de liaison complémentaire.

10. Méthode selon l'une quelconque des revendications précédentes dans laquelle le ou chaque composé marqueur inerte est lié à son membre de liaison complémentaire respectif présent sur une colonne d'immunoaffinité.

11. Méthode selon l'une quelconque des revendications précédentes dans laquelle chaque membre de liaison complémentaire respectif est un anticorps monoclonal spécifique pour son composé marqueur inerte.

FIG.1

| | MOLECULAR WEIGHT |
|---|---|
| Aflatoxin $G_2$ | 330 |
| Aflatoxin $G_1$ | 314 |
| Zearalenone | 318 |
| Zearalenol | 320 |
| Zearalanol | 322 |
| Aflatoxin $B_1$-N-acetyl-lysine | 516 |

FIG.2

FIG. 3

FIG.4